# EUROPEAN PATENT APPLICATION

(11) **EP 1 818 406 A1**
(43) Date of publication of application: **15.08.2007**
(21) Application number: 05791013.5
(22) Date of filing: 26.08.2005
(51) Int. Cl.: C12N 15/85, A61K 31/713, A61P 31/00

(54) **GENE CONSTRUCTION, VECTOR AND DNA VACCINE FOR THE VACCINATION OF AQUATIC ANIMALS**

(30) Priority: 27.08.2004 ES 200402093
(71) Applicant: Instituto Nacional De Investigacion y Tecnologia agraria y Alimentaria (INIA), 28040 Madrid (ES)
(72) Inventor: COLL MORALES, Julio, E-28040 Madrid (ES)
(74) Representative: Gonzalez Palmero, Fé
(86) International application number: PCT/ES2005/000475
(87) International publication number: WO 2006/035084

(57) **Abstract**

The gene construct comprises a nucleic acid sequence that encodes an immunogenic peptide of an aquatic animal pathogen under the control of an expression control sequence that can direct the expression of said immunogenic peptide in an aquatic animal and which comprises the sequence of a cytomegalovirus (CMV) promoter joined to an expression-enhancing sequence at position 5'. The vector comprises said gene construct and can be used to produce DNA vaccines that can be used to prevent infectious diseases in aquatic animals and is of particular interest in aquaculture. Said vaccines can be used for the mass vaccination of aquatic animals using an immersion/sonication process.

## Description

### FIELD OF THE INVENTION

The invention relates, in general, to the prevention of infectious diseases (viral, bacterial and parasitic) in aquatic animals, which is of particular interest in aquaculture, by using DNA expression systems that comprise the sequence encoding immunogenic peptides of aquatic animal pathogens. The invention also relates to the administration of said DNA expression systems to said aquatic animals, in particular, with the simultaneous vaccination of a plurality of aquatic animals (mass vaccination) using said DNA expression systems.

### BACKGROUND OF THE INVENTION

The growth of intensive aquaculture has led to an increase in the incidence of fish disease caused by viruses, bacteria or parasites. Prevention methods using vaccines are one of the most commonly used means of controlling these diseases. However, at present it has only been possible to control a few diseases caused by bacteria (redmouth, furunculosis and vibriosis) using traditional vaccines (inactivated bacteria). However, thanks to the application of new recombinant DNA technologies to the production of protein antigens directly in the host or DNA vaccines, it is expected that innovative products will be developed in this field.

Traditionally, vaccines have been classified into live (attenuated) and dead (inactivated) vaccines. The dangers of these traditional vaccines are reversion to virulence and the residual percentage of live pathogen, respectively. The advent of recombinant DNA technology has enabled the construction of live vectors (innocuous bacteria or viruses) that can transport an antigen of interest to protect against a pathogen, or the external production of said antigen isolated from the pathogen (protein subunit vaccines) or the internal production of the antigen isolated from the pathogen (DNA vaccines). Both cases avoid the need to handle large quantities of pathogen. Subunit vaccination for controlling infections in fish consists of using genetic engineering to produce subunits of the infectious agent instead of the complete infectious agent. To do this, plasmid DNA vectors from bacteria that encode the protein subunits of the infectious agent are obtained by genetic engineering. These vaccines are only effective if applied by intramuscular injection with adjuvants, fish by fish.

Some of the methods currently used for administering vaccines are not technically or economically practical. For example, the direct injection of a vaccine, fish by fish, is an intensive and expensive task in relation to the market value of the fish species. Moreover, the needles used can cause cross-infection, meaning that an accidental injection in a human can lead to serious infection or an anaphylactic reaction. An easier and cheaper method of administering a viral or bacterial vaccine is by oral administration, where the vaccine is directly added to the water or incorporated into the fish food. Methods have therefore been developed for the mass vaccination of fish by immersion in solutions containing DNA vaccines.

Patent document US 5780448 includes a review of the use of DNA vaccines for vaccinating fish. Said patent discloses a composition for inducing an immune response in a fish, which comprises an expression vector with an expression control sequence that can direct the expression in fish of an immunogenic polypeptide, such as the cytomegalovirus (CMV) promoter or a sequence comprising the CMV promoter and CMV intron A at position 3' in relation to the CMV promoter, and (ii) a DNA-encoding sequence that encodes an immunogenic peptide of a fish pathogen, such as the G protein (pG) of viral haemorrhagic septicaemia virus (VHSV) in rainbow trout. The vaccination methods disclosed in said patent include the method of vaccination by immersion. However, said patent makes no mention of the possibility of applying ultrasound to the bath to increase the efficacy of mass vaccination by immersion.

One of the new technologies with the greatest possibilities for application in the field of vaccines is immunisation using transient-expression recombinant plasmids or DNA vaccines, which require more effective eukaryotic vectors. When using DNA vaccines, it is not necessary to handle live viruses or assemble viral particles, and it is possible to select peptides that are relevant to the protection of each individual animal that is vaccinated. A suitable conformation of pathogenic proteins is used, as they are produced in the same host as the natural infection. This method can also be used with several genes at the same time.

To produce DNA vaccines it is necessary to develop efficient vectors that can correctly express the appropriate immunogenic peptide. However, most of the eukaryotic proteins expressed in bacteria are incorrectly folded and therefore present low specific activities compared to the native proteins. This is what occurs in the case of viral proteins such as the pG of viral hemorrhagic septicaemia virus (VHSV) [Estepa et al., 1994] or parasitic proteins. This is due to the fact that the production of completely active eukaryotic proteins requires post-transductional modifications such as correct formation of the disulphide bridges, glycosylation, phosphorylation, correct assembly of monomers, proteolytic processing or functional transport to membranes, all of which are processes that cannot be carried out by bacterial cells. Vectors for the expression of eukaryotic proteins in eukaryotic cells include prokaryotic sequences to aid the propagation of the plasmid in bacteria and eukaryotic sequences for performing transcription (sequences upstream of 5', promoters, intron-processing signals) and translation (polyadenylation signals) in eukaryotic cells.

Therefore, there is still a need to develop alternatives to the existing systems for vaccinating aquatic animals and protecting them against different diseases. Said systems should preferably be easy and economical to produce and administer, and they should induce a strong long-term immunity without the need to administer a subsequent booster. Said systems should preferably be suitable for stress-free application to aquatic animals and it should be possible to use them for mass vaccination of aquatic animals in order to facilitate their administration and to minimise their application costs.

### SUMMARY OF THE INVENTION

The invention relates to the immunisation of aquatic animals against infection caused by viral, bacterial or parasitic pathogens through the use of DNA expression systems (vectors) that comprise an enhancing 5' sequence, a promoter and a nucleic acid sequence that encodes an immunogenic peptide of an aquatic animal pathogen. The invention also relates to the administration of said DNA expression systems to said aquatic animals. In a particular and preferred embodiment, said vectors are administered by subjecting the aquatic animals to a process of immersion-sonication in a bath containing said vectors, thus allowing the mass vaccination of aquatic animals.

More specifically, it has now been found that the use of a promoter, such as a promoter selected from a promoter that can direct the expression of a peptide in mammal cells, such as the cytomegalovirus (CMV) promoter, bound to its expression-enhancing sequence at position 5', allows a more efficient expression of immunogenic peptides of aquatic animal pathogens in aquatic animal cells than that achieved using the CMV promoter. Said enhancer-promoter combination can be used to generate gene constructs and vectors for developing DNA vaccines. It has also been observed that it is possible to simultaneously vaccinate a plurality of aquatic animals through a process of immersion-sonication and that the integrity of said vectors is not altered after successive sonication cycles, which means that they can be reused in different mass vaccination cycles of aquatic animals.

Therefore, one aspect of the invention relates to a gene construct that comprises an expression control sequence that can direct the expression of an immunogenic peptide in an aquatic animal, and a nucleic acid sequence that encodes an immunogenic peptide of an aquatic animal pathogen. Said expression control sequence consists of 5' sequences that enhance the expression of the cytomegalovirus (CMV) promoter and the CMV promoter itself.

Another aspect of the invention relates to a vector that comprises said gene construct. Said vector can be used to produce a vaccine to protect aquatic animals against aquatic animal pathogens, which is an additional aspect of this invention.

Another aspect of the invention relates to a vaccine comprising said gene construct or said vector, and, optionally, one or more adjuvants and/or pharmaceutically acceptable vehicles. In a particular embodiment, said vaccine is a DNA vaccine. Said vaccine can be used to protect aquatic animals from viral, bacterial or parasitic infections.

Another aspect of the invention relates to the use of said gene construct or said vector to produce a vaccine for administering to aquatic animals through a process comprising the immersion of said aquatic animals in a bath containing said vaccine and the sonication of said bath containing said aquatic animals and said vaccine.

Another aspect of the invention relates to a method for simultaneously administering a vaccine to a plurality of aquatic animals that comprises the immersion of said plurality of aquatic animals in a bath containing said vaccine and the sonication of said bath containing said aquatic animals and said vaccine.

### BRIEF DESCRIPTION OF THE FIGURES

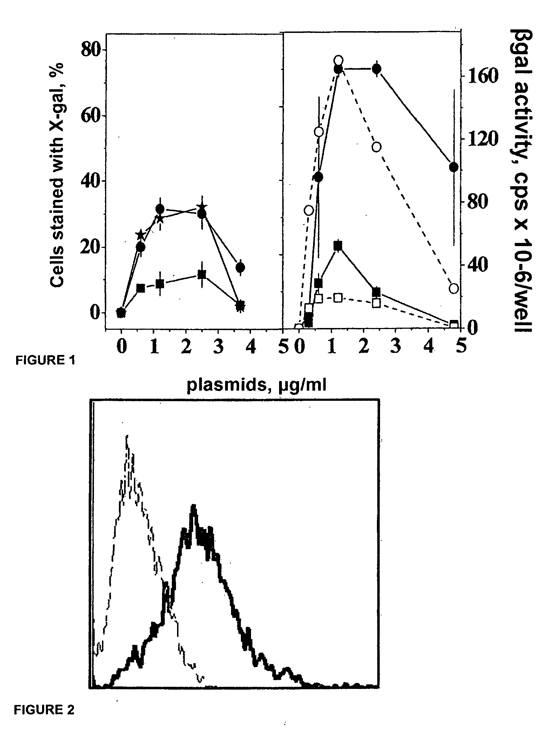

- Figure 1: is a graph showing the percentage of EPC stained by Xgal with plasmids with different lengths of the CMV promoter. Approximately 500,000 EPC cells (cell line from *Epithelioma Papullosum Cyprini)* per ml were transformed with 0.6 µg of plasmids complexed with 2 µl of fugene in 100 µl of serum-free medium. The two experiments with the most extreme results (left and right) show the range of values found in 8 experiments. The means and standard deviations of duplicate results are shown. [Coloured squares (■) : cells stained with Xgal obtained with pCMVß; coloured stars: cells stained with Xgal obtained with pMOKßgal ; coloured circles (•) : cells stained with Xgal obtained with pMVC1.4βgal ; empty squares (□): βgal activity in cps obtained with pCMVß ; and empty circles (o): βgal activity in cps obtained with pMCV1.4.
- Figure 2: is a graph showing expression of the pG of VHSV in the membrane of pMCV1.4-G-transfected EPC cells stained with anti-pG antibodies (Abs) and analysed by FACS. The EPC cells were not transfected (grey) or were transfected with the pMCV1.4-G plasmid (black).

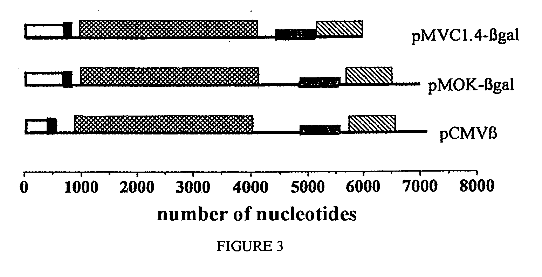

- Figure 3: schematically shows maps of pMCV1.4βgal, pMOKBal and pCMVβ. The black box represents the minimum CMV promoter; the white boxes to the left of said black boxes represent the 5' regions; the box represents the βgal gene; the striped box represents the antibiotic-resistant gene; and the remaining box represents the bacterial plasmid regions that are common to the three plasmids.

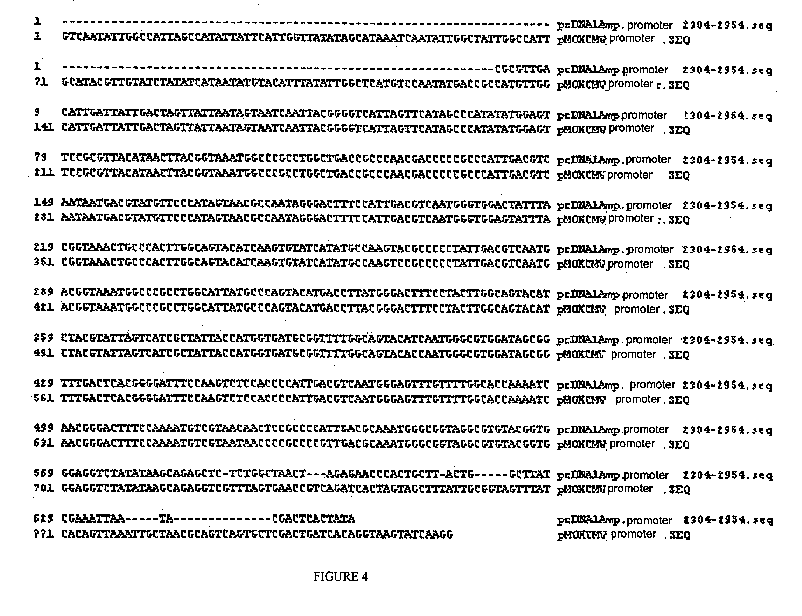

- Figure 4: shows a comparison of the cytomegalovirus (CMV) promoter sequences used by the original pQBI25 and pcDNAI/Amp vectors and the improved pMOK/pMCV1.4 vector containing the enhancing 5' sequences. As can be seen in the figure, vectors pQBI25 and pcDNAI/Amp used in previously published studies are missing sequences at both the 5' terminal end and the 3' terminal end of the long CMV promoter contained in the new plasmids. The long CMV promoter of the pMOK and pMCV1.4 vectors is the same. The conservation of increased transfection efficiency in the pMCV1.4 vector, from which bacterial sequences were removed, shows that the greater efficiencies of pMOK and pMCV1.4 compared to pQBI25 are due to differences in the length of the sequences of the CMV promoter included in each vector.

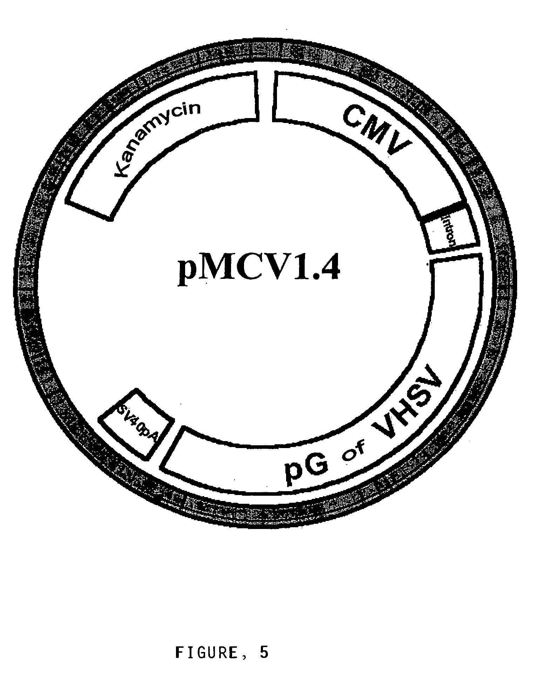

- Figure 5: shows the pMCV1.4G plasmid included in the gene construct that comprises the long pCMV promoter operatively joined to a gene of interest, which, in this specific case, is the G protein of viral haemorrhagic septicaemia virus.

### DETAILED DESCRIPTION OF THE INVENTION

The invention provides, in general, methods and compositions for immunising aquatic animals against infection caused by viral, bacterial or parasitic pathogens. Basically, the nucleic acid sequence that encodes an immunogenic peptide of an aquatic animal pathogen is introduced into said animal, said immunogenic peptide thereby being expressed in the animal's cells and thus inducing an immune response that protects against infection caused by said pathogen.

Therefore, one aspect of the invention relates to a gene construct, hereinafter the gene construct of the invention, which comprises an expression control sequence that can direct the expression of an immunogenic peptide in an aquatic animal, and a nucleic acid sequence that encodes an immunogenic peptide of an aquatic animal pathogen, wherein said expression control sequence is a nucleotide sequence comprising an A sequence and a B sequence, wherein said A sequence comprises an expression enhancing sequence, and said B sequence comprises the cytomegalovirus (CMV) promoter sequence, and wherein the 3' end of said A sequence is joined to the 5' end of said B sequence.

The expression control sequence in the gene construct of the invention that comprises said A and B sequences is sometimes referred to herein as "long CMV promoter" to distinguish it from other control sequences that comprise the CMV promoter but lack said expression enhancing sequence (A sequence) at position 5' in relation to the CMV promoter. This expression control sequence that only comprises the CMV promoter, which lies outside the scope of the invention, is sometimes referred to herein as "short CMV promoter".

The A sequence comprises an expression enhancing sequence. The length of said A sequence can vary within a wide range, e.g. between 100 and 1,000 base pairs, although in a particular embodiment said A sequence has a length of 218 base pairs. Practically any sequence that can enhance the expression of a peptide in an aquatic animal could be used in the gene construct of the invention. However, in a particular embodiment, said A sequence comprises, or consists of, the sequence of 218 base pairs between nucleotide 1 and nucleotide 218 of the nucleotide sequence shown in SEQ ID NO: 1.

The B sequence comprises, or consists of, the CMV promoter sequence. Said sequence is already known and is found in commercial plasmids, e.g. in the pcDNA3 plasmid (Invitrogen).

In a practical embodiment, the expression control sequence in the gene construct of the invention consists of said A and B sequences and comprises, or consists of, the sequence of 798 nucleotides shown in SEQ ID NO: 1.

The gene construct of the invention comprises a nucleic acid sequence, operatively joined to said expression control sequence that can direct the expression of an immunogenic peptide in an aquatic animal, which encodes an immunogenic peptide of an aquatic animal pathogen. As used herein, the expression "operatively joined" means that the immunogenic peptide is expressed in the correct reading frame under the control of said expression control sequence.

"Aquatic animal", as used herein, includes any multi-cellular organism that lives in water, typically fish. Preferably, said aquatic animal is an animal belonging to a fish species reared by aquaculture. Illustrative examples of said aquatic animals include teleost fish, such as vertebrate fish, e.g. salmonids (e.g., rainbow trout, salmon, etc.), carp, turbot, gilthead sea bream, sea bass, etc.

The term "immunogenic peptide of an aquatic animal pathogen", as used herein, refers to complete proteins and fragments thereof and to peptides that are epitopes (e.g. CTL epitopes), associated with viruses, bacteria or parasites that cause diseases in aquatic animals. Therefore, the nucleic acid sequence encoding an immunogenic peptide in the gene construct of the invention encodes an immunogenic peptide derived from a viral, bacterial or parasitic pathogen of an aquatic animal.

When humoral immunity is required, the nucleic acid sequence encoding the immunogenic peptide will preferably be a nucleic acid sequence that encodes the complete antigenic protein, or a relatively large fragment thereof, instead of nucleic acid sequences encoding small fragments. However, when cellular immunity is required, or when humoral immunity could be harmful to the aquatic animal, the nucleic acid sequence encoding the immunogenic peptide will preferably be a nucleic acid sequence that encodes a relatively small fragment of the corresponding antigenic protein (CTL epitopes).

In a particular embodiment, the gene construct of the invention comprises a nucleic acid sequence that encodes an immunogenic peptide of a viral pathogen, such as a novirhabdovirus, of an aquatic animal. For example, said peptide can be the G protein (pG) or the nucleoprotein (N) of viral haemorrhagic septicaemia virus (VHSV) in rainbow trout; the pG or the N of infectious haematopoietic necrosis virus (IHNV); the VP1, VP2, VP3 proteins or the structural N protein of infectious pancreatic necrosis virus (IPNV); the pG of spring viraemia of carp (SVC); etc.

In another particular embodiment, the gene construct of the invention comprises a nucleic acid sequence that encodes an immunogenic peptide of a bacterial pathogen of an aquatic animal, such as some proteins of *Renibacterium,* e.g. protein p57 of *R. salmoninarum.* Additional illustrative examples of said immunogenic peptides of bacterial pathogens are cited in US 5780448.

Furthermore, in another particular embodiment, the gene construct of the invention comprises a nucleic acid sequence that encodes an immunogenic peptide of a parasitic pathogen of an aquatic animal, such as the surface antigens of *Ichthyophthirius* (US 5780448).

The gene construct of the invention can contain two or more nucleic acid sequences that encode two or more immunogenic peptides of one or more aquatic animal pathogens. In this case, the expression control sequence in the gene construct of the invention can direct the expression of said nucleic acid sequences encoding said immunogenic peptides, provided that they are in the same reading frame to express a fusion protein. This situation could occur, for instance, for expressing the VP2 and VP3 proteins of IPNV as a fusion protein. The gene construct of the invention can be obtained by using techniques that are widely known in the state of the art (Sambrook et al., 1989).

The gene construct of the invention can preferably be found in a suitable vector. Therefore, another aspect of the invention relates to a vector, hereinafter the vector of the invention, which comprises at least one gene construct of the invention. Said vector can be a viral vector or a non-viral vector. In general, the choice of vector will depend on the host cell in which it is subsequently to be introduced. The vector of the invention can be obtained using conventional methods known to a person skilled in the art (Sambrook et al., 1989).

In a particular embodiment, the vector of the invention is a non-viral vector, such as a DNA plasmid or an expression vector that can be expressed in eukaryotic cells, e.g. in animals cells, which comprises at least one gene construct of the invention, which, when introduced into a host cell, is either integrated into the genome of said cell or not.

The vector of the invention can also contain the necessary elements for expression of the immunogenic peptide and the elements that regulate its transcription and/or translation. The vector of the invention can also contain RNA-processing sequences such as intron sequences for transcript splicing, transcription termination sequences, sequences for peptide secretion, etc. If desired, the vector of the invention may contain an origin of replication and a selectable marker, such as an antibiotic-resistant gene.

In one particular embodiment, the vector of the invention contains a single gene construct of the invention. However, in another particular embodiment, the vector of the invention contains two or more gene constructs of the invention. In this case, the vector of the invention can encode two or more different immunogenic peptides of the aquatic animal pathogen. Alternatively, the vector of the invention can encode two or more different immunogenic peptides that can induce an immune response against two or more different aquatic animal pathogens, thereby producing a multi-purpose vaccine. For example, as well as the pG of VHSV, the VP2 of IPNV could be included in the same reading frame.

When the vector of the invention comprises two or more gene constructs of the invention, the transcription of each nucleic acid sequence encoding each immunogenic peptide can be directed from its own expression control sequence to which it is operatively joined or it can be two or more peptides in the same reading frame.

When the vector of the invention is introduced into cells of an appropriate aquatic animal, the cells into which said vector of the invention has been introduced express the immunogenic peptide in the vector of the invention, resulting in the immunisation of the aquatic animal against the pathogen in question, as said immunogenic peptide triggers an immune response reaction in the aquatic animal. It is thus possible to immunise aquatic animals against different pathogens without the need to use attenuated live or inactivated vaccines, but simply by using the sequences encoding the corresponding immunogenic peptides incorporated into suitable vectors. The vector of the invention therefore forms the basis for a DNA vaccine and can be used to immunise aquatic animals that can be infected by different pathogens. Therefore, another aspect of the invention relates to the use of a vector of the invention to produce a vaccine for protecting animals against aquatic animal pathogens. In a particular embodiment, said vaccine is a DNA vaccine.

Another aspect of the invention also relates to a vaccine, hereinafter the vaccine of the invention, which comprises an effective amount of a gene construct of the invention, or of a vector of the invention, and, optionally, one or more adjuvants and/or pharmaceutically acceptable vehicles. In a particular embodiment, the vaccine of the invention is a DNA vaccine.

As used herein, the term "vaccine" refers to a material that can produce an immune response. The vaccine of the invention can therefore immunise aquatic animals against diseases caused by pathogens of such aquatic animals. Furthermore, as is known, the activation of CTL (cytotoxic T lymphocytes) activity resulting from the in vivo synthesis of the immunogenic peptide could produce immunity against said diseases, not only from a prophylactic but also from a therapeutic point of view, particularly after the development of the disease in aquatic animals at aquaculture installations.

The vaccine of the invention can be used to protect aquatic animals that can be infected by aquatic animal pathogens. In a particular embodiment, said pathogens are pathogens of aquatic animals reared in aquaculture installations. Non-limiting examples of said pathogens include VHSV, VHNV, IPNV, the virus that causes spring viraemia of carp (SVCV), *Aeromonas salmonicida, Renibacterium salmoninarum, Yersinia, Pasteurella* (including *piscicida), Vibriosis* (including *anguillarum* and *ordalii), Edwardsiella* (including *ictaluri* and *tarda), Streptococci, Ichthyophthirius,* etc.

In a particular embodiment, the gene construct of the invention is incorporated into a vector of the invention. To do this, the vaccine of the can be prepared in the form of an aqueous solution or suspension, in a pharmaceutically acceptable vehicle, such as saline solution, phosphate buffered saline (PBS), or any other pharmaceutically acceptable vehicle.

In another particular embodiment, the gene construct of the invention, or the vector of the invention, can be incorporated into conventional transfection reagents, such as liposomes, e.g. cationic liposomes, fluorocarbon emulsions, cochleates, tubules, gold particles, biodegradable microspheres, cationic polymers, etc. A review of said transfection reagents can be found in US 5780448. Said transfection reagents that comprise the gene construct of the invention, or the vector of the invention, are an additional aspect of the invention.

In the meaning used herein, the expression "effective amount" refers to an effective amount for the treatment and prevention of diseases caused by aquatic animal pathogens.

The pharmaceutically acceptable vehicles that can be used in the formulation of a vaccine of the invention must be sterile and physiologically compatible, e.g. sterile water, saline solution, aqueous buffers such as PBS, alcohols, polyols and suchlike. Said vaccine may also contain other additives, such as adjuvants, stabilisers, antioxidants, preservatives and suchlike. The available adjuvants include, but are not limited to, aluminium salts or gels, carbomers, nonionic block copolymers, tocopherols, muramyl dipeptide, oil emulsions, cytokines, etc. The amount of adjuvant that should be added depends on the nature of the adjuvant. The stabilisers available for use in vaccines according to the invention are, e.g. carbohydrates, including sorbitol, mannitol, dextrin, glucose and proteins such as albumin and casein, and buffers such as alkaline phosphatase. The available preservatives include, among others, thimerosal, merthiolate and gentamicin.

The gene construct of the invention, or the vector of the invention, can include CpG dinucleotides, as they have immunostimulatory effects, thus enabling the DNA to act as an adjuvant.

The vaccine of the invention can be administered by any appropriate route of administration that results in an immune response to protect against the pathogen in question, for which said vaccine will be formulated in a manner that is suitable for the chosen route of administration. Although the vaccine of the invention can be administered orally, intramuscularly, by particle bombardment or by spraying using conventional methods (US 5780448) for the simultaneous immunisation of a large number of aquatic animals, the preferred method is to submerge said aquatic animals in a solution comprising the vaccine of the invention. To do this, the vaccine of the invention can be prepared in the form of an aqueous solution or suspension, in a pharmaceutically acceptable vehicle, such as saline solution, phosphate buffered saline (PBS), or any other pharmaceutically acceptable vehicle.

The vaccine of the invention can be prepared using conventional methods known by a person skilled in the art. In a particular embodiment, said vaccine is prepared using the mixture, if applicable, of a vector of the invention, optionally having one or more adjuvants and/or pharmaceutically acceptable vehicles.

Different assays performed by the inventors have shown that very good results are achieved when the aquatic animals to be immunised are immersed in a bath comprising a vaccine of the invention and ultrasound is applied to said bath containing said vaccine and said aquatic animals. Ultrasound can be applied simultaneously or subsequent to immersion of the aquatic animals in said bath. Although the inventors do not wish to be bound to a particular theory, it is thought that ultrasound application (sonication) opens up gaps and/or pores between the cells and tissues and/or in the cell membranes of the epithelia of aquatic animals, thus enabling the vaccine of the invention to penetrate into the aquatic animal in a way that is non-traumatic and stress-free for the aquatic animal.

Another aspect of the invention therefore relates to the use of a gene construct of the invention or a vector of the invention to produce a vaccine for administering to aquatic animals through a process comprising the immersion of said aquatic animals in a bath containing said vaccine and the sonication of said bath containing said aquatic animals and said vaccine.

Furthermore, another aspect of the invention relates to a method for simultaneously administering a vaccine to a plurality of aquatic animals (mass vaccination) that comprises the immersion of said plurality of aquatic animals in a bath containing said vaccine and the sonication of said bath containing said aquatic animals and said vaccine. In a particular embodiment, said vaccine is a vaccine of the invention.

The conditions in which the animals are immersed in the bath containing the DNA solution and the sonication conditions (pulse distribution, frequencies, power per unit of surface area, etc.) may vary within a wide range depending on numerous factors. However, in a particular embodiment, the animals may be immersed in the bath containing the DNA solution (vaccine, e.g. vaccine of the invention) for between 1 and 30 minutes, preferably about 10 minutes, and the sonication may last for 8 to 24 seconds, distributed in four or more consecutive pulses depending on the size of the animal (2 seconds per pulse for animals measuring 2-3 cm and 6 seconds per pulse for animals measuring 5-7 cm), the maximum ultrasound frequency that is applied must be 40 kHz at between 0.4 and 0.6 Watts/cm2. A higher power of ultrasound has damaging effects on the animals and less power has no effect at all. The animals are then removed from the bath and taken to other tanks (aquariums) where they are left to develop immunity to the pathogen in question.

Assays performed by the inventors have shown that the practical difficulty of injecting a vaccine into a large quantity of aquatic animals, one by one, can be solved by using sonication (low power ultrasound) as a method of introducing the vector that constitutes the DNA vaccine. The intrinsic efficacy of the improved vector with the new design increases the practical possibilities of its application. The reuse of the vector for several immersions also increases its potential practical application.

Vaccination using a DNA vaccine that comprises the gene construct of the present invention induces an immune response against an antigen expressed in vivo by introducing its gene into the animal, which has the following advantages:
In vivo synthesis of the antigen, which enables DNA immunisation against all pathogens whose virulence and antigenic responses are due to a protein; this involves the generation of specific cytotoxic responses by presenting the processed antigen without the risk of generating a true infection (in this regard, it is similar to attenuated live vaccines, but without the reversion-associated problems of these);
Continual expression of the antigen, continual expression of the antigen for at least several days, which, in the animal models tested, means a strong and lasting cell-mediated and humoral immune response without the need for a booster; in aquatic animals, and particularly in fish, it must be taken into account that this only occurs at the correct temperature, and only when the immune system is mature;
Cell-mediated immunity, viruses and many bacterial pathogens (e.g. *Renibacterium* and *Aeromonas)* are intracellular pathogens and it is difficult to produce antibodies against them to neutralise them, which means that they require cell-mediated immunity for maximum protection (DNA vaccines are the best solution in these cases);
Adjuvant activity of DNA, the presence of cpG dinucleotides repeated in bacterial DNA but absent in eukaryotic DNA has immunostimulatory effects, which can be increased by adding the CpG dinucleotide to the vector; and
Economical and practical advantages, DNA vaccines are inexpensive and easy to manufacture; they are stable in terms of temperature and can be used in any species at any age. It is also possible to produce multi-purpose vaccines against several pathogens in the same or in different plasmids.

In the specific case of VHSV, it is not possible to achieve complete protection using recombinant fragments of the pG expressed in prokaryotic vectors, such as *Escherichia coli* and *Yersinia ruckeri* or *Aeromonas salmonicida.* The best protection achieved to date has been with the recombinant pG of VHSV expressed in yeast or in baculoviruses but with very low specific activity. The use of DNA vaccines does not involve handling live viruses or viral assembly, and it is possible to select peptides that are relevant to the protection of each individual animal.

Overall, the invention makes it possible to reduce the amount of vector that is needed to immunise aquatic animals by optimising the vectors (by selecting from more efficient promoters) and the methods by which the vector is transmitted (by reusing the vector solution). In this regard, one advantage of the mass vaccination method provided by this invention lies in the fact that the vaccine of the invention can be reused two or more times, since repeating the sonication process up to three times did not alter the integrity of the gene construct of the invention. The amount of the gene construct of the invention that can be used in the vaccine of the invention depends on numerous factors, including the aquatic animal to be treated and its characteristics (age, size, weight, general state, etc.), the route of administration, stability and immunogenic peptide activity, etc. However, although the inventors do not wish to be bound by any particular dosages that are mentioned below, it is thought that for oral immunisation between 0.1 and 50 µg of the gene construct of the invention is typically required per aquatic animal for several days. For immunisation by IM or IP injection, between 0.1 and 10 µg of the gene construct of the invention is typically required per aquatic animal. For immunisation by spraying, a volume of 1 ml containing between 0.1 and 10 mg/ml of the gene construct of the invention is typically required per aquatic animal. Aquatic animals immunised by the immersion-sonication method of the present invention may be incubated in a bath typically containing between 1 and 10 µg /ml of an aqueous solution of the gene construct of the invention in a minimum volume that is sufficient for the aquatic animals to survive for the short period of time (a few seconds) that is needed in order for them to take up the gene construct of the invention and for the immune response to take place in the aquatic animal. A typical amount for immunising aquatic animals by particle bombardment is between 10 ng and 1 µg.

The following examples illustrate the invention and must not be considered to limit its scope.

### EXAMPLE 1

### Development of an improved DNA vector for use-reuse in a DNA vaccination method by immersion-sonication in fish. Application to VHSV in rainbow trout

### I. MATERIALS AND METHODS

### Detection of GFP expression

This assay was used to detect cells transfected with plasmids encoding GFP that were expressing the protein. The EPC cell monolayers were examined and photographed with an inverted Nikon microscope with ultraviolet illumination using a DM510 B-2A blue filter block and a SBIG ST-7 camera (Santa Barbara Instrument Group, Santa Barbara, CA, USA).

A filter was used to avoid unspecific infrared fluorescence (eyeguard for CFWN 10X MB J99000 ND4). The CCDOPS program, version 4.03 (Santa Barbara Instrument Group) was used to take and process the microphotographs under the microscope (Fernández-Alonso, 2000; Fernández-Alonso et al., 1999; Fernández-Alonso et al., 2001).

### Detection of ß-galactosidase (βgal) expression

The Gal-screen assay (Tropix, Bedford, MA, USA) was used to determine βgal activity in transfected EPC cells with maximum sensitivity. After removing the supernatant from the culture that covered the transfected EPC cell monolayers, 200 µl of Triton 0.025% x100 per well were added and it was stirred for 15 minutes to bring about lysis. 100 µl per well of a mixture of substrate and lysis-enhancer buffer were then added and 30 minutes later, aliquots of 20 µl were counted for 2 seconds each in a MiniLumat LB9506 apparatus (EG&G Berthold, Postfach, Germany). In the conditions used here, 1 pg of βgal yielded 4x10⁴ cps (López et al., 2001).

### Histological staining of βgal

To view βgal activity in transfected EPC cells, the EPC monolayers were fixed for 5 minutes with methanol at -20°C and they were stained with 2 mg/ml of 5-bromo-4-chloro-3-indolyl-ß-D-galactopyraniside (Xgal) in 5 mM ferrocyanide II, 5 mM ferrocyanide III and 2 mM MgCl₂ in PBS. After 16 hours, the monolayers were washed with water and air-dried. To estimate the percentage of stained cells, three photographs were randomly taken using an inverted Olympus microscope with a digital photographic camera. 700-1,000 cells were counted per well and the results were expressed in means and standard deviations of two wells per experiment (López et al., 2001).

### Viral Haemorrhagic Septicaemia Virus (VSHV)

The strain of viral haemorrhagic septicaemia virus (VSHV) used here was French isolate VHSV-07.71 (Bernard et al., 1983; DeKinkelin and LeBerre, 1979; LeBerre et al., 1977), which was obtained from rainbow trout *Oncorhynchus mykiss* (Walbaum). The virus was produced and titrated according to the method described by DeKinkelin (DeKinkelin and LeBerre, 1979). First removing the medium used to culture the cells, EPC monolayers were infected with VHSV in a medium with 2% bovine foetal serum (BFS), at low multiplicity of infection (MOI=10⁻²-10⁻⁴). The cells were kept in adsorption with the virus for 1 hour at 14°C, stirring gentle every 15 minutes and flasks were filled with medium with 2% SFB, until complete lysis of the cells was achieved (10-14 days). The inocula for neutralisation were obtained by separating the cell debris from the supernatant by freezing and defrosting, then centrifuging at 3000 rpm, recovering the supernatant and freezing at -70°C. As regards purification of the virus, the method followed here is basically that described by DeKinkelin (DeKinkelin, 1972), modified by Basurco (Basurco, 1990). After centrifuging to discard the cell debris of infected EPC cell cultures, the virus was precipitated with 7% polyethylene glycol 6.000 (PEG-6.000) in 2.3% ClNa at 4°C, stirring for 2 hours. After centrifuging at 10000 g for 45 minutes, the sediment was resuspended in 1-2 ml of TNE (0.15 M Tris, 0.15 mM NaCl, 1 mM EDTA, pH 7.6). To increase the purity, the virus was ultracentrifuged using a 15-45% sucrose gradient in TNE at 80000 g for 270 minutes in a Beckman 25-75 ultracentrifuge using a SW27 rotor. The quantity of protein was estimated by measuring the absorbance at 280 nm [A₂₈₀ₙₘ (∈ = 1.4)] or by staining with Coomassie blue and comparing with known standards, taking the detection limit as 1 µg per band of protein detected.

### Plasmids used

The plasmids used were mainly pCMVß (7.2 kbp) (Clontech Labs, Palo Alto, CA, USA), pMOKBgal (6.9 kbp) or pMCV1.4ßgal (5.9 kbp) (Ready Vector, Madrid, Spain). All of them contained the ß-galactosidase (βgal) gene of *E.* coli under the control of the minimum early cytomegalovirus (CMV) promoter. However, whilst pCMVß only contains the minimum promoter (100 bp) and 687 bp of enhancer sequences (minimum promoter + 687 bp 5' = short promoter), plasmids pMOK βgal and pMCV1.4ßgal contain 218 bp of additional enhancer sequences, from nucleotide (nt) 1 to nt 218 of SEQ ID NO: 1, at position 5' in relation to the CMV promoter. Plasmids pMOK βgal and pMCV1.4ßgal contain the promoter identified herein as the "long CMV promoter" whose 798-nt sequence is shown in SEQ ID NO: 1. The pMCV1.4ßgal plasmid is derived from the pMOKßgal plasmid by eliminating approximately 1 kbp of unnecessary bacterial sequences and by including a multiple cloning site.

Other promoters were assayed with βgal such as the short CMV promoter, which is a commercial product in plasmids pcDNAI/Amp and pcDNA3 (InVitroGen), the SV40 promoter (pβgal control, provided by Clontech) and the carp actin promoter (pAE6, provided by Dr Estepa, University of Elche, and ßactin-lacZ, provided by Dr McLean, University of Southampton).

Green fluorescent protein (GFP) and luciferase (Luc) were assayed as alternative marker genes. The green fluorescent protein (GFP) gene used was cloned under the short CMV promoter in the GFP-PQBI₂₅ plasmid (6.2 kbp) (Quantum Biotech Inc, Montrevil-sous-bois, France) or under the carp actin pGFPQBI₂₅-pAE6 promoter that contains the GFP gene. The Luc gene used was cloned under the CMV promoter using commercial plasmids pGL3ctrl, pRL-CMV and pRLnull (Promega, USA) and pCMV-Luc provided by Dr Brémont (INRA, Institut National de la Recherche Agronomique, Paris, France).

The G3-pcDNAI/Amp construct, which encodes the pG of VHSV, was generated by taking the G-pcDNAI construct (Dr Michel Brémont, INRA, France) that contains the pG gene of VHSV (French isolate 07.71) cloned in the pcDNAI vector (4.0 kbp) (Invitrogen) and sub-cloning it in commercial vector pcDNAI/Amp (4.8 kbp) (Invitrogen) (Fernández-Alonso et al., 1999). It was also sub-cloned under the carp actin promoter (G-pAE6 provided by Dr Estepa, Miguel Hernández University (UMH), Spain) and the T7 promoter (G-pGEMTeasy provided by Dr Brémont, INRA, France).

### Sub-cloning of plasmid pGEMTeasy-G into plasmid pMCV1.4

In order to test expression of the pG of VHSV in plasmids with the long CMV promoter, the pG obtained in pGEMTeasy had to be sub-cloned into the pMCV1.4 plasmid, which was what most efficiently expressed βgal in EPC. To do this, the pG gene of VHSV was obtained by means of a preparative digestion with 2 µg of the pGEMTeasy-G construct (Dr Brémont, INRA, France) with *EcoRI* (10 U/µl) (GibcoBRL, Postfach, Germany), as the pG is cloned between two *EcoRI* sites, for 2 hours at 37°C in Techne dri-block apparatus. Likewise, the pMCV1.4 (Ready Vector) was linearised. To reduce its religation, first the *EcoRI* of the digested pMCV1.4 plasmid was inactivated at 65°C for 15 minutes and then alkaline phosphatase (Roche, Barcelona, Spain) was added. The mixture was incubated at 37°C for 60 minutes and then the phosphatase was inactivated at 65°C for 15 minutes. The digestion products were separated in a low melting point (LMP) 1% agarose gel (Sambrook et al., 1989), extracting and purifying the bands obtained using columns of the SNAP commercial kit (InVitroGen, Barcelona, Spain). The plasmid and the pG insert were ligated (plasmid: insert ratio of 1:3, including controls without the insert) at room temperature for 2 hours using T4 DNA ligase (Roche) with a final volume of 20 µl per ligation mixture. The 20 µl of ligated mixtures were pipetted into the wells of 96-well plates on MF filters (Millipore, Madrid, Spain) and they were dialysed for 45 minutes, floating the plates on distilled water. 10 µl of Top 10 competent E. *coli* bacteria (InVitroGen, Barcelona, Spain) were then transformed by electroporation with 1 µl of ligation mixture dialysed in 0.1 cm cuvettes (Biorad) at 1.8 kV in an Eppendorf 2.510 electroporator. After transformation, 0.3 ml of TB medium (Sigma) was added with 8 ml of glycerol/1, pH 7.5 and the bacteria were incubated at 37°C for 1 hour in an incubator with a stirring mechanism. 9 cm Petri plates were seeded with 200 µl of TB-kanamycin per plate (100 µg/ml) and were incubated overnight at 37°C.

The plasmid DNA was extracted by the CTAB method using 6 transformed colonies and a ligation control colony. To do this, cultures of 2 ml of TB-ampicillin medium (100 µg/ml) with the colonies in 13 ml tubes (Starstedt, Inc., Newton, USA) were incubated overnight at 37°C with a stirring mechanism. The bacterial precipitate was recovered by centrifuging at 12000 rpm for 10 seconds and it was subjected to lysis with STET solution (50 mM Tris-CIH pH 8, 50 mM EDTA pH 8, 8% sucrose, 0.1% Triton x100) and lysozyme (1 mg/ml) for 2 minutes at room temperature and for 45 seconds in a heating block at 100°C. It was then centrifuged at 12000 rpm for 8 minutes and the supernatant was recovered and treated with RNase (50 µg/ml) for 30 minutes at 37°C. The DNA was treated for 5 minutes at room temperature with a previously-solubilised 5% CTAB solution (Sigma) (0.2% final volume) and was centrifuged at 12000 rpm for 10 minutes. The recovered precipitate was segregated from the CTAB with 100% ethanol and 5 M ClNa at a temperature of -70°C for 20 minutes and it was washed with 70% cold ethanol. The precipitate was resuspended in sterile distilled H₂O (Sigma).

To detect the presence of the insert, the DNA extracted from the different clones was digested for 2 hours at 37 °C with *EcoRI.* The products of digestion in a 1% agarose gel were separated and the clones of the insert were selected. To distinguish the direction of the insert, the isolated plasmids were subjected to digestion with *SacI* and *XmaI* enzymes that produced fragments of 4.3+0.08 kbp if the direction of the insert was correct or 1.6+2.8 kbp if the direction of the insert was otherwise. One of the clones of the insert in the right direction was chosen and it was amplified for subsequent experimentation.

### Characterisation by PCR and sequencing

The published 1.58-kbp gene sequence of the pG of VHSV containing the G-pcDNAI and pGEMTeasy-G starting construct (Thiry et al., 1991a; Thiry et al., 1991b) was used for preliminary characterisation of the constructs. 4 oligonucleotides were designed in order to amplify two overlapping fragments in the frg#11 region of the pG gene (nucleotides (nt) 13 to 339 and nt 184 to 1,536 of the pG sequence). The oligonucleotide sequence in the 5' → 3' direction was as follows:
#59 (forward): ATG GAA TGG AAC ACT TTT TTC TTG G TG;
#60 (reverse): GGT GAGC TCG ATA AGT CAC TCT GTG CAG;
#61 (forward): CGA CCA GCT CAA CTC AGG TGT CCT CAT;
#62 (reverse): TCA GAC CGT CTG ACT TCT GGA GAA CTG.

The TM was calculated manually and using the PCGene program. The expected size of the amplified fragments was 327 (oligos #59 and #60, nt 13 to 339 of the G sequence) and 1, 353 (oligos #61 and #62, nt 184 to 1,536 of the VHSV G gene sequence) base pairs. Amplifications were performed by polymerase chain reaction (PCR) with each of the two pairs of oligonucleotides (#59-#60 and #61-#62) and the two G gene constructs, the G-pcDNAI starting construct and the sub-cloned G3-pcDNAI/Amp, including negative controls without DNA and with the commercial plasmid used in the sub-cloning (pcDNAI/Amp). AmpliTaq DNA Polymerase (Perkin-Elmer, Bucks, UK) was used. For the reaction, 5 U of Ampli Taq was mixed with 200 ng of each of the oligonucleotides of the corresponding pairs, 10 ng of plasmid DNA, the corresponding buffer, in the presence of 2 mM MgCl₂ and 0.2 mM of dNTPs mixture (Promega), in a final volume of 100 µl. First, 30 amplification cycles were carried out for 1 minute at 95°C, 1 minute at 55°C and 2 minutes at 72°C, followed by a final extension cycle of 10 minutes at 72°C. 10 µl of each PCR were then made to migrate in a 1% agarose gel for 2 hours at 80 V to check the amplification.

### Plasmid purification

Plasmids were used to transform E. *coli DH5alpha* bacteria (Clontech, Madrid, Spain). A large amount of plasmids were then prepared using the commercial DNA purification system Wizard Plus Megaprep (Promega, Madison, WI, USA), following the manufacturer's instructions, and a modified purification protocol based on alkaline lysis omitting the final resin-based chromatography step. The protocol was simplified and optimised with regard to performance and degree of purity. The optimised protocol is as follows: 3 g of bacteria paste was resuspended in 250 ml tubes (GSA rotor) with 40 ml of solution I (50 mM Tris-Cl, 40 mM EDTA, 100 µg/ml of RNaseA, pH 7.5) and was used for less than 20 minutes, mixing by inversion until a transparent medium was obtained with a lysis solution II (0.2 N NaOH, 0.1% sodium dodecyl sulphate (SDS)). The bacterial debris was then precipitated with a neutralisation solution III (1.3 M potassium acetate, pH 4.8) and the precipitate was removed by centrifuging at 14000 g for 15 minutes and filtered through filter paper. To recover plasmid from the supernatant, it was precipitated with 55 ml of isopropanol for 5 minutes, stirring by inversion, and was centrifuged at 14000 g for 15 minutes. After it was dried and dissolved in H₂O (Sigma), the DNA was precipitated again in 40 ml tubes (SS34 rotor) with 0.25 mM sodium acetate, pH 5.2, and 100% cold ethanol and lastly it was washed with 70% ethanol. After the precipitate was vacuum-dried for 20 minutes or overnight at room temperature, the DNA was dissolved in H₂O (Sigma). Finally, the total amount of DNA was estimated by measuring the absorbance at 260 nm in a photospectrometer (A₂₆₀ₙₘ) and the percentage of plasmid was estimated by agarose gel electrophoresis at 1% and the plasmid solutions were adjusted to 0.5-1 mg/ml of total DNA (A₂₆₀ₙₘ). The plasmid preparations contained 80-100% of plasmid DNA, as was shown by agarose gel electrophoresis, the rest being other bacterial DNA.

### Assays with 2-cm immunoincompetent trout and GFP-pQBI₂₅

The GFP-PQBI₂₅ plasmid that encodes the green fluorescent protein (GFP) was used for these assays. The low-frequency ultrasound-based assays were carried out on groups of 5 trout weighing 0.3-0.5 g (2 cm) in a glass vessel with 20 ml of aquarium water inside a 250 ml bath sonicator at an ultrasound power of 40 W/cm and a frequency of 40 kHz (Selecta, Barcelona, Spain) filled with 100 ml of water. First the ultrasounds were applied in two 2-second pulses with a 1-minute interval between pulses, then GFP-PQBI₂₅ was added and 2 1-second pulses were applied with a 1-minute interval between pulses. The trout were then kept with the GFP-PQBI₂₅ for 30 minutes, they were transferred to 500 ml were kept at 4-10°C with aeration for 1-2 weeks while GFP expression was analysed.

### In vivo vaccination/retest assays with immunocompetent trout and G3-pcDNAI/Amp

The G3-pcDNAI/Amp plasmid that encodes the VSHV G protein was used for these assays. They were performed using trout of a size and weight between 1-5 g (5-7 cm), which came from the Fuentehermosa (Puebla Lillo, León, Spain) and El Molino (Manzanares del Real, Madrid, Spain) fish farms. Until the moment of the vaccination/retest, the trout were kept in 30-litre closed-circuit aquariums. The installations where the testing was carried out are located at the High Security Laboratory of the National Institute of Agrarian Research (I.N.I.A. - Instituto Nacional de Investigaciones Agrarias) in Valdeolmos (Madrid).

The vaccination/retest experiments were carried out by bath-immersion following previously described protocols (Basurco, 1990). Each experiment included an aquarium with 10 µg of G3-pcDNAI/Amp injected per trout and a control aquarium with unvaccinated trout. Briefly, the trout were kept in a volume of 500 ml of aquarium water with strong aeration through diffusers at 8-10°C for 30-120 minutes after the vaccines were added, the temperature and time varying for different experiments. Immediately after this, the trout were put back in the aquarium, waiting for 30 days before giving the retest with VSHV. In all the cases the mixture and bath were added to the aquariums.

For the vaccination using low-frequency ultrasounds, 20-30 trout measuring 5-7 cm were kept in a bath sonicator with 100 ml of aquarium water (total volume of approximately 250 ml) with the plasmid formulations. Two 10-second pulses were applied, the G3-pcDNAI/Amp was added and two 2-second pulses were applied. There was a 1-minute interval between pulses. They were then kept in containers with a total volume of 500 ml of aquarium water for 30 minutes with aeration and then they were transferred to 30-litre closed-circuit aquariums at 10°C, until the moment of the retest.

The experiments on vaccination by injection to study promoters were carried out in the same conditions as the other experiments, by direct intramuscular injection of the plasmids. Plasmids G-pcDNA3-CMV and pcDNA3 were used as a negative control.

The retests by immersion were carried out at a dose of 10 plaque-forming units per ml (PFU/ml) of the 07.71 strain of VSHV. The retests by intramuscular (IM) injection and oral administration were carried out at a dose of 106 PFU/ml of virus 50-200 µl per trout. Aeration with diffusers was used when the retests were performed in 0.5-1 litres in recipients outside the aquariums. After the retests were performed, the mortality data were collected for 30-40 days and the relative survival rate (RSR) was calculated using the formula: 1 - mortality of trout treated with plasmid/mortality in the control with unvaccinated trout x 100.

### II. RESULTS

### Analysis of possible alterations in the G3-pcDNAI/Amp vector after sonication

For vaccination using low-frequency ultrasound, 20-30 trout measuring 5-7 cm were kept in a bath sonicator with 100 ml of aquarium water (total volume of approximately 250 ml) with the plasmid formulations. Two 10-second pulses were applied, the G3-pcDNAI/Amp was added and two 2-second pulses were applied. There was a 1-minute interval between pulses. They were then kept in containers with a total volume of 500 ml of aquarium water for 30 minutes with aeration and then they were transferred to 30-litre closed-circuit aquariums at 10°C, until the moment of the retest. Samples of the vector solution in 100 ml of water were taken before and after sonication and the sonication process was repeated 1-3 times. The analysis of the DNA by agarose gel electrophoresis showed that there were no significant alterations in either the amount or the migration of the plasmids used and that they can therefore be used for more than one sonication.

### Studies of DNA vaccination by injection of G-pcDNA3 with short CMV promoters

4 experiments were carried out, involving vaccination by injection and a retest with the G-pcDNA3 plasmid.

In the first of the experiments, the retest was performed by immersion. The mortality rate in the group injected with G-pcDNA3 was 26%. As the mortality of the unvaccinated control groups was between 85-90%, the CMV promoter was capable of protecting the trout.

In the second of the experiments, the retest was carried out by intraperitoneal (IP) injection, trout by trout. In this case the mortality rate of the unvaccinated control groups was 68% and no significant differences were found between the mortality rates in trout injected with G-pcDNA3-CMV (38%) or pcDNA3 (34.8%).

In the third experiment, two aquariums were used per group and the retest was carried out by immersion. In this case the mortality rate for the unvaccinated control groups was 52% and 70%, and for the groups vaccinated with pcDNA3 it varied between 10% and 48% from one aquarium to the other. This is an illustrative example of the variations between aquariums that affect this type of experiments. The mortality rates of trout injected with G-pcDNA3-CMV (15%) were lower as a whole than those achieved in the controls, confirming that the CMV promoter can express pG and protect trout.

In the last of the two experiments described, the mortality rates observed were slower than usual for this infection (50% mortality in 15-17 days compared to 7-9 days). To test whether this delay was due to the low dose of virus that was given (10 PFU/ml), the dose administered was multiplied by 100 and another experiment was performed with 10 µg of plasmids (a dose ten times higher than in previous experiments), as was a retest with an IM dose and another administered orally. This large dose of virus in the retest caused 50% mortality after 3-5 days in the unvaccinated controls. The mortality rate for the controls (injected with PBS and pcDNA3) was 100% and 95%, respectively. In the groups vaccinated with G-pcDNA3-CMV (72%) the mortality rate of the trout was somewhat less, but nonetheless very high. The fact that the mortality rate is so high could be due to an excess of inoculum. However, a 5-day delay in mortality was observed between the unvaccinated control group and the trout vaccinated with pcDNA3 and G-pcDNA3-CMV.

### DNA vaccination by immersion in G3-pcDNAI/Amp with sonication

Only ultrasound, DOTAP (dioleoyl trimethyl ammonium propane) and bactofection (immersion in recombinant bacteria containing the plasmid) were tested in subsequent experiments, methods by which the best results were achieved in previous experiments with GFP-pQBI₂₅. The lowest mortality rates corresponded to the group of trout vaccinated with G3- pcDNAI/Amp+ultrasound (approximately 50%) and the highest to trout vaccinated with E. *coli* (G3-pcDNAI/Amp) (93.3%), G3-pcDNAI/Amp+DOTAP (95%) and the combination of formulations (100%) [G3-pcDNAI/Amp + DOTAP + ultrasound] and (85.7%) [G3-pcDNAI/Amp + bactofection + ultrasound] (data not shown).

In a second experiment the trout vaccinated by immersion in G3-pcDNAI/Amp+ultrasound again presented the lowest relative mortality rate (47.6%), compared to those vaccinated by G3-pcDNAI/Amp+bactofection (57.1%) and G3-pcDNAI/Amp+DOTAP (87.5%). The mortality rate of trout injected with G3-pcDNAI/Amp was 15.5%.

The relative survival rate calculated from the three experiments using vaccination by immersion and sonication, compared to the mortality rates of unvaccinated trout, is shown in Table 1. The maximum relative survive rate of 71.5% was achieved by injection of G3-pcDNAI/Amp. Of the methods assayed by immersion, the application of G3-pcDNAI/Amp+ultrasound to the trout was the method that provided the greatest survival rate in the retest (50.1%). Immersion in G3-pcDNAI/Amp+DOTAP (12.3%) and immersion in E. *coli* (G3-pcDNAI/Amp) (20.7%) and G3-pcDNAI/Amp (8.8%) achieved RSRs very similar to the overall mortality rate. Furthermore, a greater reproducibility for different experiments (a very difficult fact to obtain in experiments of vaccination/retest with fish) was observed in the case of G3-pcDNAI/Amp+ultrasound (CV=3.2%), than with G3-pcDNAI/Amp+DOTAP and E. *coli* (G3-pcDNAI/Amp) (CV=10.9 and 14.0%, respectively).

**Table 1**

| **Relative survival rate (RSR) of trout 1 month after vaccination by immersion in G3-pcDNAI/Amp** | |
|---|---|
| Immersion method | RSR |
| **G3-pcDNAI/Amp+injection** | **71.5 ± 0.0 (n=1)** |
| **G3-pcDNAI/Amp+ultrasound** | **50.1 ± 3.2 (n=3)** |
| G3-pcDNAI/Amp | 8.8 ± 0.0 (n=2) |

Approximately 20 trout, measuring 5-7 g per trout, were used for each part of the experiment. A control aquarium with unvaccinated trout was included in each of the three experiments. The mortality rate in the unvaccinated aquariums was 100%, 87.5% and 63.6%, respectively, for the three experiments. The means and standard deviations were calculated for the number of experiments (indicated in brackets). The significant results are highlighted in bold.

### Increased efficiency of transfection through the use of vectors with the long CMV promoter

Figure 2 shows that the pMOKßgal plasmid and its derivative with lower molecular weight, the pMCV1.4ßgal plasmid, but both with the long CMV5 promoter, stained 3-4 times more transfected EPC cells with Xgal than the pCMVß plasmid. Increases in the efficiency of transfection were even greater when βgal activity was estimated by luminescence (approximately 10 times greater, i.e. corresponding to approximately 170 x 106 cps, equivalent to 3400 ng of ßgal). No significant differences were found between the transfection efficiencies of pMOKßgal and its derivative with a lower molecular weight pMCV1.4ßgal (n=8). However, the absolute value of efficiency varied from experiment to experiment. As a result of the greater efficiency of transfection, visual inspection of the transfected EPC plates by pMOKßgal/pMCV1.4ßgal and stained with Xgal, showed a blue colour that could never have been achieved with pCMVß.

The βgal activity achieved with pMOKßgal/pMCV1.4ßgal was also optimum at 1-3 µg pf plasmid per ml. Higher or lower DNA concentrations reduced βgal activity to background levels, confirming the results previously achieved with pCMVβ (López et al., 2001).

These results were confirmed by staining EPC monolayers transfected with pMCV1.4βgal and stained with Xgal. The cells with a large amount of βgal are stained very bright blue, whilst other cells are stained light blue. The number of stained cells remained constant for several days before spreading into other adjacent cells after a week.

Table 2 shows a summary of the transfection efficiencies and their reproducibility achieved throughout 57 experiments over about 3 years of experimentation. The results achieved show that, using pCMVβ (short CMV promoter), 12.8±6.5% transfected EPC cells (n=24) was obtained with a coefficient of variation (CV) of 54.1%. The use of pMOKβgal or pMCV1.4βgal, plasmids that use a longer CMV promoter than pCMVβ, increased the estimated transfection efficiency for the number of EPC cells stained with X-gal by 2.2-2.4 times (to approximately 30%), and reduced the CV to approximately 40%. It should be highlighted that, although the number of transfected EPC cells increases two-fold, the amount of βgal expressed per cell can be ten times higher.

**Table 2**

| **Transfection efficiencies and reproducibility using plasmids with different promoters and cell treatments** | | | | |
|---|---|---|---|---|
| **Plasmid** | **Treatment** | **Cells stained with Xgal, % (no. of experiments)** | **CV, %** | **ITE** |
| pCMVß | --- | 12.8 ± 6.5 (24) | 54.1 | 1 |
| pMOKßgal | --- | 29.1 ± 12.3 (6) | 42.2 | 2.2 |
| pMCV1.4ßgal | --- | 30.7 ± 12.1 (13) | 39.3 | 2.4 |

The transfection efficiencies of pcDNAI/Ampßgal and pQBI25 GFP were similar to those of pCMVß. CV, coefficient of variation expressed in percentage. ITE, increase in transfection efficiency, which was calculated according to the formula: percentage of transfected cells / percentage of cells transfected with pCMVß.

It was later shown that the improvement in transfection did not depend on the gene that had been used under the promoter. This was done by substituting the βgal gene for the pG gene of VHSV, and expression of the pG of VHSV in the membrane was estimated by staining with specific antibodies and by the formation of syncytia. EPC cells were transfected and it was shown that the pG was expressed in the membrane in a larger number of cells and with greater intensity (Figure 2) when the pMCV1.4 with the long CMV promoter was used instead of the pcDNAI/Amp with the short CMV promoter. Moreover, the number of syncytia formed with pMCV1.4-G was greater and of a larger size (number of nuclei per syncytium) when the long CMV promoter was used (data not shown).

### Studies of the difference in sequences between the long and short CMV promoter

Figure 3 shows the differences in the sequences of plasmids pCMVß, pMOKßgal and pMCV1.4βgal, which are restricted to the lengths of their CMV promoters. Furthermore, Figure 4 shows the differences in sequences between the short CMV promoters, which are found in the pcDNAI/Amp, pcDNA3 and pQBI25 vectors, and the long CMV promoter (SEQ ID NO: 1) of the pMOKBgal and pMCV1.4βgal vectors.

### III. DISCUSSION

### Mechanism of protection in trout vaccinated with G3-pcDNAI/Amp in the retest with VSHV

DNA vaccination by immersion and the application of a series of low-frequency ultrasounds were used to immunise and protect 5-7 cm trout (the minimum size at which they can be vaccinated). Using DNA vaccination with ultrasound it has been possible to establish around 50% protection, with less variation between experiments (Table 1). This indicates that ultrasound, despite the difficulties in reproducing the results in this type of experiments, is sufficiently reproducible and that its potential practical application could be improved if the vector solutions were reused for several series of applications, thus increasing the number of vaccinated trout per quantity of vector.

The mechanisms by which ultrasound can induce transfection in trout are not known. At a macroscopic level, it can be observed that mucus covering the gills and the surface of the trout's body is released into the aqueous medium when ultrasound is applied. The removal or reduction of the physical barrier formed by the mucus surrounding the whole surface of the trout could facilitate access of the DNA to the skin cells. However, it is not known whether DNA reached the epithelial cells of the trout through the gills and from the system circulatory or directly from the water.

At cell level, the interaction of low frequency ultrasound and duration with live tissues is complex and has been studied very little in the case of fish. It is known that ultrasound penetrates well into live tissues, producing thermal and cavitational effects related to osmotic pressure, surface tension and cell adhesion. The intensity and duration of the ultrasound applied to trout in this work cannot produce hyperthermia or damage by cavitation in the outer epithelium of the skin, as its intensity and duration are below the levels that cause them. On the other hand, therapeutic ultrasound (approximately 2 W/cm² at 3000 kHz for 30 to 90 seconds) causes the intercellular space to widen and microfilaments of the desmosomes that connect adjacent cells in the carp skin epithelium to break (Frenkel and Kimmel, 2000). For the experiments mentioned herein, 0.4-0.6 W/cm² at 40 kHz were used for 8 seconds in total for 2-cm trout or 24 seconds in total for 5-7 cm trout. The ultrasound application time at said intensities was chosen as approximately half the time at which ultrasound pulses had visibly harmful effects on the trout (haemorrhages, detachment of the mucosa, or death). At the intensities and times used herein the treated trout continued to live without macroscopic alterations for the 2-3 months that the longest experiments lasted. At cell level it has also be shown that the application of ultrasound at said intensities and times to a cell suspension (EPC) did not reduce the adhesion capacity or viability of the cells (data not shown).

The ultrasound application conditions differed from detailed experiments carried out in other studies, in terms of the surface area of the bath, the existence of glass barriers (in the case of 2-cm trout), the distance from the transduction surface to the trout (varied between 0-8 cm) and the angle of ultrasound application. These last two variations are due to the fact that the trout were not immobilised, as was the case with experiments in other studies, but were free to swim around the ultrasound bath.

Since the perforation of the intercellular space described in cell membranes requires higher ultrasound intensities (2.2 W/cm² at 3000 kHz for 30 to 90 seconds), it is unlikely that said effect is related to the uptake of the plasmid DNA by the trout skin cells in our experiments, but more experimental evidence is needed to clarify this possibility. This perforation could be temporary and/or could vary with the power applied, the duration of the pulses, the time between pulses, the moment when the DNA is added and small variations in the temperature.

There are many variables in an ultrasound treatment protocol and only a few have been studied to date. All these variables have been kept constant in our experiments, but by studying them it might be possible to optimise the amount of DNA that reaches the inside of the cells with a greater expression of the G protein to be presented to the immune system, thereby inducing a more efficient immune response in the retest.

### Possible application of vaccination by immersion using ultrasound in aquaculture

Vaccination by immersion currently offers great potential for application in aquaculture, as it is an effective and practical method for the mass vaccination of fish. Most commercial bacterial vaccines tend to be administered by this method. It is therefore of great practical interest to continue studying DNA vaccination along these lines, in view of the promising protection results using ultrasound.

Although the use of low-frequency ultrasound could easily be scaled for practical use in fish farms, the amount of plasmid required seems to be excessive for it. The optimum concentration of DNA was approximately 10 µg/ml. In order to minimise said quantity, it has been shown that it is possible to reuse the plasmid solution as it is not degraded with at least 3 series of sonications and, furthermore, to make it more efficient. The fact that the DNA in the bath in which the trout were kept immersed after ultrasound treatment remains unaltered could direct future assays towards reusing the same plasmid solutions to vaccinate several groups of trout in the same bath, within a limit of time and use, which would have to be studied. The results of this work open up a new approach to improve fish vaccination methods.

### Improvement of the promoter in the plasmids used

Another way of increasing DNA transfection was by assaying the influence of several promoters on the transfection efficiency. The CMV promoter always achieved better transfection efficiencies than the SV40 promoter (data not shown). However, although all commercial CMV promoters have a minimum of 100 bp, they differ in the length of the 5' sequences (Figure 4). Assays using 2 plasmids with CMV promoters (pCMVß and pMOKßgal) of different lengths showed a 2- to 3-fold increase in the number of transfected cells when the plasmid with the longest 5' sequences (pMOKβgal) ["long CMV promoter" (SEQ ID NO: 1)] was used. In some experiments the increase in transfection made it possible to view blue monolayers. Moreover, βgal expression estimated by luminescence could increase by as much as 10-fold (Figure 1), suggesting that the use of the long CMV promoter not only increased the percentage of cells that expressed βgal but also the expression for each transfected cell.

A smaller derivative of pMOKßgal, the so-called pMCV1.4ßgal, was also capable of increasing transfection efficiency. As this derivative also contained a multiple cloning site, it was the plasmid chosen to sub-clone the pG of VHSV to demonstrate that the increase achieved did not depend on the gene under the control of the promoter. By comparing the sequences of the 3 plasmids (Figure 3), it can be concluded that the essential difference lies in the additional 218 bp of the CMV promoter in plasmids pMOKβgal and pMCV1.4βgal. Neither the size (pMOKβgal is the same size as pCMVβ) nor the bacterial sequences that are common to the 3 plasmids are responsible for the differences observed. The use of these additional 218 bp, either with the short CMV promoter or with other fish promoters, can serve to optimise vectors for their use in DNA vaccines, both by injection (Anderson et al., 1996b; Lorenzen et al., 1998) and by immersion-sonication (Fernández-Alonso et al., 1999, Fernández-Alonso, 2000; Fernández-Alonso et al., 1998; Fernández-Alonso et al., 2001).

### BIBLIOGRAPHY

Anderson, E.D., Mourich, D.V. and Leong, J.C. (1996b). Gene expression in rainbow trout (Onchorynchus mykiss) following intramuscular injection of DNA. Molecular Marine Biology Biotechnology 5:105-113.
Basurco, B. (1990). Estudio, identificación y caracterización del virus de la septicemia hemorrágica vírica en España. PhD Thesis. Universidad Complutense de Madrid. 1:178.
Bernard, J., LeBerre, M.B. and DeKinkelin, P. (1983). Viral haemorrhagic septicaemia of rainbow trout: relation between the G polypeptide and antibody production of fish after infection with the F25 attenuated variant. Infect.Immun. 39:7-14.
DeKinkelin, P. (1972). Le virus D?gtved.II-Purification. Armáis Recherche Vétérinary 3:199-208.
DeKinkelin, P. and LeBerre, M. (1979). Mass virus production in fish cell system. Dev.Biol.Stand. 42:99-104.
Estepa, A. (1992). Estudios de immunización con proteínas electroeluidas y clonadas del virus de la septicemia hemorrágica vírica de la trucha. University of Madrid Spain Doctoral Thesis PhD Thesis:243.
Fernández-Alonso, M. (2000). Expresión en vectores eucariontes de genes para vacunación DNA por inmersión en el modelo trucha/rabdo virus. PhD Thesis, Universidad de Navarra, Pamplona, Spain PhD Thesis: 169p.
Fernandez-Alonso, M., Alvarez, F., Estepa, A., Blasco, R. and Coll, J.M. (1999). A model to study fish DNA inmersion-vaccination by using the green fluorescent protein. J.Fish Dis. 22:237-241.
Fernández-Alonso, M., Álvarez, F., Estepa, A. and Coll, J.M. (1998). Vacunas DNA en Acuicultura. AquaTIC 4 : http ://aquatic.unizar .es/N 1 /art401/DN A_vac.htm.
Fernández-Alonso, M., Rocha, A. and Coll, J.M. (2001). DNA vaccination by immersion and ultrasound to trout viral haemorrhagic septicaemia virus. Vaccine 19:3067-3075.
LeBerre, M., De Kinkelin, P. and Metzger, A. (1977). Identification sérologique des rhabdovirus des salmonidés. Bull.Off.Int.Epiz. 87:391-393.
López, A., Fernandez-Alonso, M., Rocha, A., Estepa, A. and Coll, J.M. (2001). Transfection of epithelioma cyprini (EPC) carp cells. Biotechnology Letters 23:481-487.
Lorenzen, N., Lorenzen, E., Eoner-jensen, K., Heppell, J., Wu, T. and Davis, H. (1998). Protective immunity to VHS in rainbow trout (Oncorhynchus mykiss, Walbaum) following DNA vaccination. Fish Shellfish Immunol. 8:261-270.
Sambrook, J., Fritsch, E.F. and Maniatis, T. (1989). Molecular cloning. A laboratory manual. Cold Spring Harbour Laboratory Press
Thiry, M., Lecoq-Xhonneux, F., Dheur, L, Renard, A. and Kinkelin, D. (1991a). Molecular cloning of the m-RNA coding for the G protein of the viral haemorrhagic septicaemia (VHS) of salmonids. Journal Veterinary Microbiology. 23:221-226.
Thiry, M., Lecoq-Xhonneux, F., Dheur, L, Renard, A. and Kinkelin, D.(1991b). Sequence of a cDNA carrying the glycoprotein gene and part of the matrix protein M2 gene of viral haemorrhagic septicaemia virus, a fish rhabdovirus. Biochim.Biophys. Acta 1090:345-347.

### LIST OF SEQUENCES

<110> National Institute of Agrarian and Food Research and
Technology (INIA - Instituto Nacional de Investigación y
Tecnología Agraria y Alimentaria)
<120> Gene construct, vector and DNA vaccine for the
vaccination of aquatic animals
<160> 1
<170> PatentIn version 2.0
<210> 1
<211> 798
<212> DNA
<213> Artificial sequence
<220>
<223> Cytomegalovirus (CMV) promoter sequence from
nucleotides 1 to 798 containing a CMV expression-enhancing
sequence joined to the 5′ end (nucleotides 1 to 218)

GTCAATATTG GCCATTAGCC ATATTATTCA TTGGTTATAT AGCATAAATC AATATTGGCT 60
ATTGGCCATT GCATACGTTG TATCTATATC ATAATATGTA CATTTATATT GGCTCATGTC 120
CAATATGACC GCCATGTTGG CATTGATTAT TGACTAGTTA TTAATAGTAA TCAATTACGG 180
GGTCATTAGT TCATAGCCCA TATATGGAGT TCCGCGTTAC ATAACTTACG GTAAATGGCC 240
CGCCTGGCTG ACCGCCCAAC GACCCCCGCC CATTGACGTC AATAATGACG TATGTTCCCA 300
TAGTAACGCC AATAGGGACT TTCCATTGAC GTCAATGGGT GGAGTATTTA CGGTAAACTG 360
CCCACTTGGC AGTACATCAA GTGTATCATA TGCCAAGTCC GCCCCCTATT GACGTCAATG 420
ACGGTAAATG GCCCGCCTGG CATTATGCCC AGTACATGAC CTTACGGGAC TTTCCTACTT 480
GGCAGTACAT CTACGTATTA GTCATCGCTA TTACCATGGT GATGCGGTTT TGGCAGTACA 540
CCAATGGGCG TGGATAGCGG TTTGACTCAC GGGGATTTCC AAGTCTCCAC CCCATTGACG 600
TCAATGGGAG TTTGTTTTGG CACCAAAATC AACGGGACTT TCCAAAATGT CGTAATAACC 660
CCGCCCCGTT GACGCAAATG GGCGGTAGGC GTGTACGGTG GGAGGTCTAT ATAAGCAGAG 720
GTCGTTTAGT GAACCGTCAG ATCACTAGTA GCTTTATTGC GGTAGTTTAT CACAGTTAAA 780
TTGCTAACGC AGTCAGTG 798

## Claims

1. A gene construct that comprises an expression control sequence that can direct the expression of an immunogenic peptide in an aquatic animal, and a nucleic acid sequence that encodes an immunogenic peptide of an aquatic animal pathogen, wherein said expression control sequence is a nucleotide sequence comprising an A sequence and a B sequence, wherein said A sequence comprises an expression enhancing sequence, and said B sequence comprises the cytomegalovirus (CMV) promoter sequence, and wherein the 3' end of said A sequence is joined to the 5' end of said B sequence.

2. Construct according to claim 1, wherein said A sequence consists of the 218-base-pair sequence between nucleotide 1 and nucleotide 218 of the nucleotide sequence shown in SEQ ID NO: 1.

3. Construct according to claim 1, wherein said B sequence consists of the CMV promoter sequence.

4. Construct according to claim 1, wherein said expression control sequence comprises the nucleotide sequence shown in SEQ ID NO: 1.

5. Construct according to claim 1, wherein said immunogenic peptide of an aquatic animal pathogen is selected from complete proteins and fragments thereof, and peptide epitopes associated with viruses, bacteria or parasites that cause diseases in aquatic animals.

6. Construct according to claim 5, wherein said immunogenic peptide of an aquatic animal pathogen is selected from the G protein (pG) of viral haemorrhagic septicaemia virus (VHSV) in trout; the pG of infectious haematopoietic necrosis virus (IHNV); the VP1, VP2 or VP3 proteins or the structural N protein of infectious pancreatic necrosis virus (IPNV); the pG of spring viraemia of carp (SVC); the p57 protein of *Renibacterium salmoninarum;* and surface antigens of *Ichthyophthirius.*

7. Construct according to claim 1, which comprises two or more nucleic acid sequences that encode two or more immunogenic peptides of one or more aquatic animal pathogens.

8. A vector that comprises a gene construct according to any of claims 1 to 7.

9. Vector according to claim 8, selected from a viral vector and a non-viral vector.

10. Vector according to claim 8, which comprises a single gene construct according to any of claims 1 to 7.

11. Vector according to claim 8, which comprises two or more gene constructs according to any of claims 1 to 7.

12. Use of a vector according to any of claims 8 to 10 to produce a vaccine for protecting aquatic animals against aquatic animal pathogens.

13. Use according to claim 12, wherein said vaccine is a DNA vaccine.

14. A vaccine that comprises an effective amount of a gene construct according to any of claims 1 to 7, or a vector according to any of claims 8 to 11, and, optionally, one or more adjuvants and/or pharmaceutically acceptable vehicles.

15. Vaccine according to claim 14, **characterised in that** it is a DNA vaccine.

16. Vaccine according to either of claims 14 or 15 for protecting aquatic animals that can be infected by aquatic animal pathogens selected from viral haemorrhagic septicaemia virus (VHSV) in trout, haematopoietic necrosis virus (IHNV), infectious pancreatic necrosis virus (IPNV), the virus that causes spring viraemia of carp (SVCV), *Aeromonas salmonicida, Renibacterium salmoninarum, Yersinia, Pasteurella, Vibriosis, Edwardsiella, Streptococci,* and *Ichthyophthirius.*

17. Use of a gene construct according to any of claims 1 to 7, or a vector according to any of claims 8 to 11, to produce a vaccine for administration to aquatic animals by a process comprising the immersion of said aquatic animals in a bath containing said vaccine and the sonication of said bath containing said aquatic animals and said vaccine.

18. A method for simultaneously administering a vaccine to a plurality of aquatic animals that comprises the immersion of said plurality of aquatic animals in a bath containing said vaccine and the sonication of said bath containing said aquatic animals and said vaccine.
